# EUROPEAN PATENT SPECIFICATION

(11) **EP 3 166 574 B1**
(45) Date of publication and mention of the grant of the patent: **31.03.2021**
(21) Application number: 15819689.9
(22) Date of filing: 07.07.2015
(51) Int. Cl.: A61K 8/19, A61K 8/30, A61K 8/92, A61K 8/72, A61K 8/39, A61K 8/26, A61K 8/31, A61K 8/37, A61Q 1/06, A61K 8/60, A61K 8/64, A61K 8/81, A61Q 19/00, A61K 8/02, A61K 8/25

(54) **COSMETIC COMPOSITION BASED ON COLOR PARTICLES, STEARALKONIUM HECTORITE, POLYHYDROXYSTEARIC ACID, POLYGLYCERYL-1-PENTAISOSTEARATE AND A POLAR ACTIVATOR**
KOSMETISCHE ZUSAMMENSETZUNG ENTHALTEND FARBPARTIKEL, STEARALKONIUMHEKTORIT, POLYHYDROXYSTEARINSÄURE, POLYGLYCERYL-1-PENTAISOSTEARAT UND EINEN POLAREN AKTIVATOR
COMPOSITION COSMÉTIQUE À BASE DE PARTICULES DE COULEUR, D'HECTORITE DE STÉARALKONIUM, D'ACIDE POLYHYDROXYSTÉARIQUE, DE POLYGLYCÉRYL-1-PENTAISOSTÉARATE ET D'UN ACTIVATEUR POLAIRE

(30) Priority: 11.07.2014 US 201462023383 P; 09.12.2014 US 201462089476 P
(43) Date of publication of application: 17.05.2017
(73) Proprietor: Mary Kay, Inc., Addison, TX 75001 (US)
(72) Inventor: MENDOZA, Ricky, Addison, TX 75001 (US)
(74) Representative: Uexküll & Stolberg
(86) International application number: PCT/US2015/039386
(87) International publication number: WO 2016/007519

(56) References cited:
- EP-A1- 3 081 206
- WO-A1-2013/182238
- WO-A2-2016/176659
- DE-A1-102008 012 457
- US-A1- 2007 258 934
- US-A1- 2008 233 064
- US-A1- 2011 195 100
- US-A1- 2012 308 500
- US-A1- 2013 022 562
- Anonymous: "Pigmentary Grade Dispersions - Technical Literature", , 21 May 2014 (2014-05-21), XP055338077, Retrieved from the Internet: URL:http://www.in-cosmetics.com/__novadocu ments/65626?v=635491690330370000 [retrieved on 2017-01-24]
- CRODA: "The use of SOLAVEIL SPEXTRATM in personal care formulations", RESEARCH DISCLOSURE, KENNETH MASON PUBLICATIONS, HAMPSHIRE, UK, GB, vol. 552, no. 12, 1 April 2010 (2010-04-01), page 385, XP007139731, ISSN: 0374-4353
- ANONYMOUS: "Non-Nano Zincoxide", TECHNICAL LITERATURE REF NONNANOZNO,, 7 June 2012 (2012-06-07), pages 1-2, XP007920978,
- DATABASE GNPD [Online] MINTEL; July 2013 (2013-07), "irresistible flavor lip gloss", XP002775265, Database accession no. 2112050
- DATABASE GNPD [Online] MINTEL; August 2005 (2005-08), "Lipstick", XP002775266, Database accession no. 400098
- DATABASE GNPD [Online] MINTEL; October 2011 (2011-10), "Glamour lip gloss", XP002775267, Database accession no. 1645647
- DATABASE GNPD [Online] MINTEL; March 2012 (2012-03), "Maximum Colour Lipstick spf 18", XP002775268, Database accession no. 1751545
- DATABASE GNPD [Online] MINTEL; September 2014 (2014-09), "Audacious Lipstick", XP002775269, Database accession no. 2643171
- "Microspheres. Technical Literature ref MSp-003", KOBO PRODUCTS, INC., 26 June 2015 (2015-06-26), pages 1-2, XP055235213, Retrieved from the Internet: URL:http://www.koboproductsinc.com/Downloa ds/Kobo-Microspheres.pdf [retrieved on 2015-12-10]

## Description

### BACKGROUND OF THE INVENTION

### A. Field of the Invention

The present invention relates to lipsticks. In particular, the present invention concerns a lipstick that has the ability to color and moisturize the lips.

### B. Description of Related Art

Previous attempts at lip-based products such as lip sticks and lip glosses have either lacked substantivity, tended to dry-out and clump or crack on the lip, or lacked the ability to effectively treat or prevent lip-related conditions (e.g., dried or cracked lips, lip wrinkles, etc.). Attempts to solve these problems have led to formulations that were difficult to spread on the lips, had unpleasant tactile properties (e.g., heavy, oily, tacky, etc.), and had limited beneficial effects for lip-related conditions. This resulted in lips that had an unpleasant aesthetic appearance and a streaky/non-uniform coloring.

Document "Pigmentary Grade Dispersions - Technical Literature",, 21 May 2014 (2014-05-21) (http:// www.in-cosmetics.com/_novadocuments/65626?v=635491690330370000) discloses pigmentary grade dispersions to be used in anhydrous or emulsion compositions, such as lipstick, comprising stearalkonium hectorite, polyhydroxystearic acid and activator propylene carbonate. MINTEL in accession no. 2112050 "irresistible flavor lip gloss" discloses a lip product based on the same ingredients. The website www.koboproducts.com discloses microspheres based on HDI/trimethylol hexyllactone crosspolymer/silica to be used in skin products; they show a "soft focus effect"

### SUMMARY OF THE INVENTION

The present disclosure overcomes deficiencies in the art by providing lipstick compositions that are substantive, aesthetically appealing, do not pool on the lips, are easy to spread, and moisturize the lips. Furthermore, the combination of ingredients provides for lipstick compositions with a uniform color and consistency.

The invention is directed to lipstick compositions comprising color particles, stearalkonium hectorite, polyhydroxystearic acid, and a polar activator wherein the polar activator is propylene carbonate, and further ingredients as defined in appended claim 1, wherein the color particles are uniformly dispersed throughout the composition. In some embodiments, the composition may comprise 0.01 to 0.1% by weight of stearalkonium hectorite, 0.001 to 0.1% by weight of polyhydroxystearic acid, and 0.001 to 0.1% by weight of a polar activator. The polar activator is propylene carbonate. The composition comprises microspheres that increase blendability of the composition. The microspheres comprise HDI/Trimethylol hexyllactone crosspolymer and silica. The composition comprises 0.5 to 15% by weight of HDI/Trimethylol hexyllactone crosspolymer and silica. The color particles comprise a colorant. The colorant is selected from Blue 1 Lake, Red 6 Lake, Red 7 Lake, Yellow 5 Lake, iron oxides, titanium dioxide, tin oxide, and Mica. In some embodiments, the composition may further comprise *Helianthus annus* (sunflower) seed oil, pentaerythrityl tetraisostearate, bis-diglyceryl polyacyladipate-2, isononyl isononanoate polyethylene, *Simmondsia chinensis* (jojoba) butter, mica, ozokerite, sucrose polysoyate and microcrystalline wax/cire microcrystalline.

In some embodiments, the composition may further comprise triisostearyl citrate. In some embodiments, the composition may comprise 7 to 13% by weight of triisostearyl citrate. In some embodiments, the composition may comprise 1 to 3% by weight of HDI/Trimethylol hexyllactone crosspolymer.

The composition comprises polyglyceryl-10 pentaisostearate, glyceryl behenate/eicosadioate, isopropyl myristate, and tocopheryl acetate. In some embodiments, the composition may comprise 2 to 10% by weight of polyglyceryl-10 pentaisostearate, 0.1 to 3% by weight of glyceryl behenate/eicosadioate, 0.1 to 3% by weight of isopropyl myristate, and 0.1 to 3% by weight of tocopheryl acetate. In some embodiments, the composition may comprise 2 to 6% by weight of HDI/Trimethylol hexyllactone crosspolymer.

In some embodiments, the composition may further comprise methylsilsesquioxane. In some embodiments, the composition comprises 9 to 13% by weight of HDI/Trimethylol hexyllactone crosspolymer.

In some embodiments, the composition may further comprise polyglyceryl-2 diisostearate/IPDI copolymer, triisostearyl citrate, DI-PPG-3 myristyl ether adipate, and hydrogenated polyisobutene. In some embodiments, the composition may comprise 8 to 15% by weight of polyglyceryl-2 diisostearate/IPDI copolymer, 6 to 12% by weight of triisostearyl citrate, 2 to 10% by weight of DI-PPG-3 myristyl ether adipate, and 2 to 8% by weight of hydrogenated polyisobutene. In some embodiments, the composition may comprise 0.5 to 3% by weight of HDI/Trimethylol hexyllactone crosspolymer.

In some embodiments, the composition may further comprise one or more additional ingredients selected from one or more colorants, bulking agents, thickening agents, emulsifiers, fragrances, moisturizing agents, conditioning agents, and/or antioxidants. In some embodiments, the composition may be a semi-solid.

The lipsticks described in any of the aspects and embodiments herein may be formulated as a cream, serum, gel, or balm. In some embodiments, the compositions and lipsticks described herein may be in the form of a semi-solid. In some embodiments, the compositions and lipsticks described herein are anhydrous. In some embodiments, the compositions and lipsticks described herein may comprise a dermatologically acceptable vehicle. The dermatologically acceptable vehicle may be any dermatologically acceptable vehicle described herein and/or known in the art. In some embodiments, the dermatologically acceptable vehicle may be an emollient.

The lipstick described herein can be applied at least once, twice, three, four, or more times a day. Once applied, the lipsticks can remain on the skin for at least 10, 20, 30, or 60 minutes, 1, 2, 3, 4, 5, 6, 7, 8, 9, 10, 11, or 12 hours, or longer. The lipsticks can also include any one of or any combination of cosmetic and/or pharmaceutical ingredients disclosed in this specification. For instance, compositions can include ingredients from at least one, two, three, four, five, six, seven, eight, nine, and/or ten of the following categories: (1) UV absorption agents; (2) moisturizing agents; (3) antioxidants; (4) structuring agents; (5) emulsifiers; (6) silicone containing compounds; (7) essential oils; (8) thickening agents; (9) preservatives; and/or (10) conditioning agents.

It is also contemplated that the viscosity of the lipstick compositions can be selected to achieve a desired result (e.g., depending on the type of composition desired, the viscosity of such composition can be from about 1 cps to well over 1 million cps or any range or integer derivable therein (e.g., 2 cps, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, cps, etc., as measured on a Brookfield Viscometer using a TC spindle at 2.5 rpm at 25° C.) 1 cps is equal to 1 mPa.s. The compositions in non-limiting aspects can have a pH of about 6 to about 9. In other aspects, the pH can be 1, 2, 3, 4, 5, 6, 7, 8, 9, 10, 11, 12, 13, or 14. In other aspects, the compositions can be sunscreens having a sun protection factor (SPF) of 1, 5, 10, 15, 20, 25, 30, 35, 40, 45, 50, 55, or more.

The lipsticks can have a cosmetically or pharmaceutically elegant feel such a non-oily, non-greasy, non-sticky, non-tacky, and/or silky feel after being applied to skin such as hand skin.

Also disclosed is a method for coloring lips, moisturizing lips, treating dried, cracked, or chapped lips or skin in the vermillion border or preventing or reducing the appearance of lip wrinkles or wrinkles in the vermillion border, or erythemic (i.e., red or inflamed skin) in the vermillion border, comprising topically applying any one of the lipsticks disclosed in this application or any one of the mixtures disclosed in this application to dried, cracked, or chapped lips or to skin in need of prevention or reduction of lip wrinkles, wherein topical application of the compositions or mixtures colors the lips, moisturizes the lips, treats dried, cracked, or chapped lips or prevents or reduces the appearance of lip wrinkles. The composition can be applied directly onto the lips or can be applied onto lips that already have lipstick or lip balm applied thereon. The lip-based compositions can also be used to increase the production of collagen and hyaluronic acid in the lips via topical application of the composition to lips in need of increased collagen production and hyaluronic acid production.

Also contemplated are kits that include any one of the compositions disclosed throughout the specification and claims. In certain embodiments, the lipstick is comprised in a container. The container can be a bottle, dispenser, or compact applicator. The container can dispense a pre-determined amount of the composition. In certain aspects, the compositions or formed into a stick and contained in an enclosing useful for maintaining the structure of the composition and for application of the composition to the lips. In other aspects, the lipstick composition is in a dispenser which comprises an applicator for applying the lipstick to the lips. The container can include indicia on its surface. The indicia can be a word, an abbreviation, a picture, or a symbol .Kits are not part of the invention as claimed.

The compositions and methods for their use can "comprise," "consist essentially of," or "consist of' any of the ingredients disclosed throughout the specification. As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

"Consisting essentially of' means that inclusion of additional ingredients in the compositions do not materially affect the beneficial properties of the compositions as lipstick compositions. For instance, if a composition "consists essentially of' color particles, stearalkonium hectorite, polyhydroxystearic acid, and a polar activator, said composition excludes any ingredients that would materially affect the beneficial properties of the compositions for coloring lips, moisturizing lips, treating dried, cracked, or chapped lips or skin in the vermillion border or for preventing or reducing the appearance of lip wrinkles or wrinkles in the vermillion border, or erythemic (i.e., red or inflamed skin) in the vermillion border.

It is contemplated that any embodiment discussed in this specification can be implemented with respect to any method or composition of the disclosure, and *vice versa.* Furthermore, compositions of the disclosure can be used to achieve methods of the disclosure.

In one embodiment, compositions disclosed herein can be pharmaceutically or cosmetically elegant or can have pleasant tactile properties. "Pharmaceutically elegant," "cosmetically elegant," and/or "pleasant tactile properties" describes a composition that has particular tactile properties which feel pleasant on the skin (*e.g*., compositions that are not too watery or greasy, compositions that have a silky texture, compositions that are non-tacky or sticky, *etc.*)*.* Pharmaceutically or cosmetically elegant can also relate to the creaminess or lubricity properties of the composition or to the moisture retaining properties of the composition.

"Vermillion border" means the normally sharp demarcation between the lip (red colored) and the adjacent normal skin. It represents the change in the epidermis from highly keratinized external skin to less keratinized internal skin. It has no sebaceous glands, sweat glands, or hair.

"Topical application" means to apply or spread a composition onto the surface of lips or keratinous tissue. "Topical skin composition" includes compositions suitable for topical application on lips or keratinous tissue. Such compositions are typically dermatologically-acceptable in that they do not have undue toxicity, incompatibility, instability, allergic response, and the like, when applied to lips or skin. Topical skin care compositions disclosed herein can have a selected viscosity to avoid significant dripping or pooling after application to skin.

"Keratinous tissue" includes keratin-containing layers disposed as the outermost protective covering of mammals and includes, but is not limited to, lips, skin, hair and nails.

The term "about" or "approximately" are defined as being close to as understood by one of ordinary skill in the art, and in one non-limiting embodiment the terms are defined to be within 10%, preferably within 5%, more preferably within 1%, and most preferably within 0.5%.

The term "substantially" and its variations are defined as being largely but not necessarily wholly what is specified as understood by one of ordinary skill in the art, and in one non-limiting embodiment substantially refers to ranges within 10%, within 5%, within 1%, or within 0.5%.

The terms "inhibiting" or "reducing" or any variation of these terms includes any measurable decrease or complete inhibition to achieve a desired result. The terms "promote" or "increase" or any variation of these terms includes any measurable increase or production of a protein or molecule (*e.g*., matrix proteins such as fibronectin, laminin, collagen, or elastin or molecules such as hyaluronic acid) to achieve a desired result.

The term "effective," as that term is used in the specification and/or claims, means adequate to accomplish a desired, expected, or intended result.

Other objects, features and advantages of the present disclosure will become apparent from the following detailed description. It should be understood, however, that the detailed description and the examples, while indicating specific embodiments of the disclosure, are given by way of illustration only. Additionally, it is contemplated that changes and modifications within the spirit and scope of the disclosure will become apparent to those skilled in the art from this detailed description.

### DESCRIPTION OF ILLUSTRATIVE EMBODIMENTS

The compositions described herein are useful for the moisturizing, coloring, and/or treating or soothing the skin and lips. The compositions described herein may also be used to impart a fragrance, shine, or protectant film to the skin or lips. In some embodiments, the compositions are formulated as lipsticks.

Embodiments of the compositions described herein comprise compositions with color particles uniformly dispersed throughout the composition. The uniform dispersion comprises a combination of stearalkonium hectorite, polyhydroxystearic acid and a poly activator. The combination of these ingredients provides for rheological control and uniform dispersion of ingredients. The invention is as defined in the appended claims.

Embodiments of the compositions described herein comprise ingredients such as HDI/trimethylol hexyllactone crosspolymer, silica, and methylsilsesquioxane. These ingredients are sold as a combination/single ingredient under the trade name BPD-500 by Kobo Products Inc. (South Plainfield, New Jersey). In particular, BDP-500 are microspheres that are said to scatter light, which diminishes the look of fine lines on the lips while also letting enough light through so that the lips have a natural visual appearance.

Glyceryl behenate/eicosadioate is a mixture of esters of glycerin with behenic andeicosanoic acids. It is used as an emollient and emulsifying agent in cosmetic compositions.

Waxes form an important group of ingredients for the manufacture of personal care products and decorative cosmetics. Chemically, waxes are complex mixtures of heavy hydrocarbons and fatty acids combined with esters. Waxes provide structure, are less greasy than other structuring agents, and are very resistant to moisture, oxidization and microbial attack.

Ozokerite or ozocerite (also referred to as earthwax or earth wax), is a naturally occurring odoriferous mineral wax or paraffin found in many localities. It is efficient at providing hardness, gel strength, and consistency to compositions. It also insures color uniformity, has emulsifying and emollient properties, and is compatible with all kinds of mineral and plant oils and waxes.

Polyethylene is a synthetic wax used in cosmetic compositions. It is a polymer of ethylene monomers and can be used as an emulsion stabilizer, film former, and/or viscosity increasing agent in cosmetic compositions.

Beeswax (Cera Alba) is a natural wax produced by bees to build the walls of their honeycomb. Beeswax is a thickening agent. It thickens the oil portion of solid products (like lipsticks), giving them structure and keeping them solid. It stiffens eye makeup products without hardening them. It is also an emollient that smooths and softens the skin.

*Euphorbia cerifera* wax or candelilla wax is a wax derived from parts of the small Candelilla shrub native to northern Mexico and the southwestern United States. In some embodiments, the wax is derived from the leaves. It is mostly used mixed with other waxes to harden them without raising their melting point.

*Copernicia cerifera* wax or carnauba wax is obtained from the Brazilian tropical palm tree, *Copernicia cerifera.* In some embodiments, it is obtained from the leaves. The wax helps to keep an emulsion from separating into its oil and liquid components and also increase the thickness of the lipid portion of the composition, giving them structure and allowing for a smooth application. Carnauba Wax is the hardest of the commercial vegetable waxes. It is a tough, amorphous, lustrous wax that varies in color from dirty yellow to brown, green or white. This wax is sometimes called Brazil Wax.

Microcrystalline wax consists of mixed refined hydrocarbons. It is less brittle and more malleable than paraffin. Microcrystalline wax has also a greater affinity to oils than paraffin and often contains 1-4% mineral oil. Because of its ability to hold high amounts of oil (more than other waxes), microcrystalline wax finds use in practically all types of cosmetics. Microcrystalline wax is particularly useful in lip balms and lipsticks to prevent "sweating"

### A. Lip-Based Formulations

It is contemplated that the compositions disclosed herein can include any cosmetic ingredient or any combination thereof described throughout this taking into account the limitations as defined in the claims. The concentrations of the any ingredient within the compositions can vary. In some embodiments, for example, the compositions can comprise, consisting essentially of, or consist of, in their final form, for example, at least about 0.0001%, 0.0002%, 0.0003%, 0.0004%, 0.0005%, 0.0006%, 0.0007%, 0.0008%, 0.0009%, 0.0010%, 0.0011%, 0.0012%, 0.0013%, 0.0014%, 0.0015%, 0.0016%, 0.0017%, 0.0018%, 0.0019%, 0.0020%, 0.0021%, 0.0022%, 0.0023%, 0.0024%, 0.0025%, 0.0026%, 0.0027%, 0.0028%, 0.0029%, 0.0030%, 0.0031%, 0.0032%, 0.0033%, 0.0034%, 0.0035%, 0.0036%, 0.0037%, 0.0038%, 0.0039%, 0.0040%, 0.0041%, 0.0042%, 0.0043%, 0.0044%, 0.0045%, 0.0046%, 0.0047%, 0.0048%, 0.0049%, 0.0050%, 0.0051%, 0.0052%, 0.0053%, 0.0054%, 0.0055%, 0.0056%, 0.0057%, 0.0058%, 0.0059%, 0.0060%, 0.0061%, 0.0062%, 0.0063%, 0.0064%, 0.0065%, 0.0066%, 0.0067%, 0.0068%, 0.0069%, 0.0070%, 0.0071%, 0.0072%, 0.0073%, 0.0074%, 0.0075%, 0.0076%, 0.0077%, 0.0078%, 0.0079%, 0.0080%, 0.0081%, 0.0082%, 0.0083%, 0.0084%, 0.0085%, 0.0086%, 0.0087%, 0.0088%, 0.0089%, 0.0090%, 0.0091%, 0.0092%, 0.0093%, 0.0094%, 0.0095%, 0.0096%, 0.0097%, 0.0098%, 0.0099%, 0.0100%, 0.0200%, 0.0250%, 0.0275%, 0.0300%, 0.0325%, 0.0350%, 0.0375%, 0.0400%, 0.0425%, 0.0450%, 0.0475%, 0.0500%, 0.0525%, 0.0550%, 0.0575%, 0.0600%, 0.0625%, 0.0650%, 0.0675%, 0.0700%, 0.0725%, 0.0750%, 0.0775%, 0.0800%, 0.0825%, 0.0850%, 0.0875%, 0.0900%, 0.0925%, 0.0950%, 0.0975%, 0.1000%, 0.1250%, 0.1500%, 0.1750%, 0.2000%, 0.2250%, 0.2500%, 0.2750%, 0.3000%, 0.3250%, 0.3500%, 0.3750%, 0.4000%, 0.4250%, 0.4500%, 0.4750%, 0.5000%, 0.5250%, 0.0550%, 0.5750%, 0.6000%, 0.6250%, 0.6500%, 0.6750%, 0.7000%, 0.7250%, 0.7500%, 0.7750%, 0.8000%, 0.8250%, 0.8500%, 0.8750%, 0.9000%, 0.9250%, 0.9500%, 0.9750%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 65%, 70%, 75%, 80%, 85%, 90%, 95%, or 99% or any range derivable therein, of at least one of the ingredients that are mentioned throughout the specification and claims. In some aspects, the percentage can be calculated by weight or volume of the total composition. A person of ordinary skill in the art would understand that the concentrations can vary depending on the addition, substitution, and/or subtraction of ingredients in a given composition.

The disclosed compositions described herein may also include various antioxidants to retard oxidation of one or more components. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof. In some embodiments, the compositions do not contain parabens.

### B. Vehicles

The compositions disclosed herein can be incorporated into all types of vehicles (*i.e.* dermatologically acceptable vehicles). Some examples of suitable vehicles include emulsions (*e.g*., water-in-oil, water-in-oil-in-water, oil-in-water, silicone-in-water, water-in-silicone, oil-in-water-in-oil, oil-in-water-in-silicone emulsions), creams, lotions, solutions (both aqueous and hydro-alcoholic), anhydrous bases (such as lipsticks and powders), gels, and ointments or by other method or any combination of the forgoing as would be known to one of ordinary skill in the art (Remington's, 1990). Variations and other appropriate vehicles will be apparent to the skilled artisan and are appropriate for use in the compositions described herein. In certain aspects, it is important that the concentrations and combinations of the compounds, ingredients, and agents be selected in such a way that the combinations are chemically compatible and do not form complexes which precipitate from the finished product.

It is also contemplated that ingredients identified throughout this specification can be individually or combinatorially encapsulated for delivery to a target area such as skin. Examples of encapsulation techniques include the use of liposomes, vesicles, and/or nanoparticles (*e.g*., biodegradable and non-biodegradable colloidal particles comprising polymeric materials in which the ingredient is trapped, encapsulated, and/or absorbed-examples include nanospheres and nanocapsules) that can be used as delivery vehicles to deliver the ingredient to skin (*see, e.g.,* U.S. Patent 6,387,398; U.S. Patent 6,203,802; U.S. Patent 5,411,744; Kreuter 1998).

### C. Cosmetic Products and Articles of Manufacture

The compositions described herein are

### D. Additional Ingredients

In addition to the combination of ingredients disclosed herein by the Applicants, the compositions can also include additional ingredients such as cosmetic ingredients and pharmaceutical active ingredients. Some examples of these additional ingredients are described in the following subsections.

### 1. Cosmetic Ingredients

The CTFA International Cosmetic Ingredient Dictionary and Handbook (2004 and 2008) describes a wide variety of cosmetic ingredients that can be used in the compositions described herein. Examples of these ingredient classes include: fragrances (artificial and natural; *e.g*. gluconic acid, phenoxyethanol, and triethanolamine), dyes and colorants (*e.g*., Blue 1, Blue 1 Lake, Red 40, Red 28 Lake, Red 27 Lake, Red 7 Lake, Red 6 Lake, Yellow 5 Lake, titanium dioxide, aluminum hydroxide, Unipure Red 6, Unipure Red 28, Unipure Red 33, Unipure Yellow OX, Unipure Yellow 5, FD&C blue 1, D&C blue no. 4, D&C green no. 5, D&C orange no. 4, D&C red no. 17, D&C red no. 6, D&C red no. 7, D&C red no. 30, D&C red no. 33, D&C violet no. 2, D&C yellow no. 10, D&C yellow no. 11, iron oxides, chromium oxides, tin oxide, ultramarines, and mica), flavoring agents (*e.g*. Stevia rebaudiana (sweetleaf) extract), bulking agents (*e.g*. silicic acid, silica, synthetic fluorphlogopite, and aluminum calcium sodium silicate) adsorbents, lubricants, solvents (*e.g*. water, hydrocarbons, hexylene glycol, isododecane, octyldodecanol, glycerin, and propylene glycol), moisturizers (including, *e.g*., emollients, humectants, film formers, occlusive agents, and agents that affect the natural moisturization mechanisms of the skin), water-repellants, UV absorbers (physical and chemical absorbers such as para-aminobenzoic acid ("PABA") and corresponding PABA derivatives, titanium dioxide, zinc oxide, *etc.*)*,* essential oils, vitamins (*e.g.* A, B, C, D, E, and K), trace metals (*e.g.* zinc, calcium and selenium), inorganic salts (*e.g.* sodium chloride, magnesium nitrate, and magnesium chloride), anti-irritants (*e.g.* steroids and non-steroidal anti-inflammatories), botanical extracts (*e.g.* aloe vera, chamomile, cucumber extract, ginkgo biloba, ginseng, and rosemary), anti-microbial agents, antioxidants (*e.g.,* BHT and tocopherol), chelating agents (*e.g.,* disodium EDTA and tetrasodium EDTA), preservatives (*e.g.,* methylparaben and propylparaben), pH adjusters (*e.g.,* malic acid, sodium hydroxide, triethanolamine, and citric acid), absorbents (*e.g*., aluminum starch octenylsuccinate, kaolin, corn starch, oat starch, cyclodextrin, talc, and zeolite), skin bleaching and lightening agents (*e.g*., hydroquinone and niacinamide lactate), humectants (*e.g.,* sorbitol, urea, and mannitol), exfoliants, waterproofing agents (*e.g.,* magnesium/aluminum hydroxide stearate), conditioning agents (*e.g*., aloe extracts, allantoin, bisabolol, ceramides, dimethicone, hyaluronic acid, and dipotassium glycyrrhizate), and film formers (*e.g*. acrylates copolymer, hydrogenated polycyclopentadiene, polyethylene, carnauba wax, HDI/trimethylol hexyllactone crosspolymer, and polyquarternium-7). Some examples of some of these ingredients are provided in the following subsections.

### a. UV Absorption Agents

UV absorption agents that can be used in combination with the compositions of the disclosure include chemical and physical sunblocks. Some examples of chemical sunblocks that can be used include para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), cinnamates (octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, *etc.*)*,* anthranilates, ethyl urocanate, homosalate, octisalate, oxtinoxate, dibenzoylmethane derivatives (*e.g*., avobenzone), octocrylene, octyl triazone, digalloyl trioleate, glyceryl aminobenzoate, lawsone with dihydroxyacetone, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidene camphor, and isopentyl 4-methoxycinnamate. Non-limiting examples of physical sunblocks include, kaolin, talc, petrolatum and metal oxides (*e.g*., titanium dioxide and zinc oxide).

### b. Moisturizing Agents

Some examples of moisturizing agents that can be used with the compositions of the disclosure include amino acids, chondroitin sulfate, diglycerin, erythritol, fructose, glucose, glycerin, glycerol polymers, glycol, 1,2,6-hexanetriol, honey, hyaluronic acid, hydrogenated honey, hydrogenated starch hydrolysate, inositol, lactitol, maltitol, maltose, mannitol, natural moisturizing factor, PEG-15 butanediol, polyglyceryl sorbitol, salts of pyrrolidone carboxylic acid, potassium PCA, propylene glycol, sodium glucuronate, sodium PCA, sorbitol, sucrose, trehalose, urea, and xylitol.

Other examples include acetylated lanolin, acetylated lanolin alcohol, alanine, algae extract, aloe barbadensis, aloe-barbadensis extract, aloe barbadensis gel, althea officinalis extract, apricot (prunus armeniaca) kernel oil, arginine, arginine aspartate, arnica montana extract, aspartic acid, avocado (persea gratissima) oil, barrier sphingolipids, butyl alcohol, beeswax, behenyl alcohol, beta-sitosterol, birch (betula alba) bark extract, borage (borago officinalis) extract, butcherbroom (ruscus aculeatus) extract, butylene glycol, calendula officinalis extract, calendula officinalis oil, candelilla (euphorbia cerifera) wax, canola oil, caprylic/capric triglyceride, cardamom (elettaria cardamomum) oil, carnauba (copernicia cerifera) wax, carrot (daucus carota sativa) oil, castor (ricinus communis) oil, ceramides, ceresin, ceteareth-5, ceteareth-12, ceteareth-20, cetearyl octanoate, ceteth-20, ceteth-24, cetyl acetate, cetyl octanoate, cetyl palmitate, chamomile (anthemis nobilis) oil, cholesterol, cholesterol esters, cholesteryl hydroxystearate, citric acid, clary (salvia sclarea) oil, cocoa (theobroma cacao) butter, coco-caprylate/caprate, coconut (cocos nucifera) oil, collagen, collagen amino acids, corn (zea mays)oil, fatty acids, decyl oleate, dimethicone copolyol, dimethiconol, dioctyl adipate, dioctyl succinate, dipentaerythrityl hexacaprylate/hexacaprate, DNA, erythritol, ethoxydiglycol, ethyl linoleate, eucalyptus globulus oil, evening primrose (oenothera biennis) oil, fatty acids, geranium maculatum oil, glucosamine, glucose glutamate, glutamic acid, glycereth-26, glycerin, glycerol, glyceryl distearate, glyceryl hydroxystearate, glyceryl laurate, glyceryl linoleate, glyceryl myristate, glyceryl oleate, glyceryl stearate, glyceryl stearate SE, glycine, glycol stearate, glycol stearate SE, glycosaminoglycans, grape (vitis vinifera) seed oil, hazel (corylus americana) nut oil, hazel (corylus avellana) nut oil, hexylene glycol, hyaluronic acid, hybrid safflower (carthamus tinctorius) oil, hydrogenated castor oil, hydrogenated coco-glycerides, hydrogenated coconut oil, hydrogenated lanolin, hydrogenated lecithin, hydrogenated palm glyceride, hydrogenated palm kernel oil, hydrogenated soybean oil, hydrogenated tallow glyceride, hydrogenated vegetable oil, hydrolyzed collagen, hydrolyzed elastin, hydrolyzed glycosaminoglycans, hydrolyzed keratin, hydrolyzed soy protein, hydroxylated lanolin, hydroxyproline, isocetyl stearate, isocetyl stearoyl stearate, isodecyl oleate, isopropyl isostearate, isopropyl titanium triisostearate, isopropyl lanolate, isopropyl myristate, isopropyl palmitate, isopropyl stearate, isostearamide DEA, isostearic acid, isostearyl lactate, isostearyl neopentanoate, jasmine (jasminum officinale) oil, jojoba (buxus chinensis) oil, kelp, kukui (aleurites moluccana) nut oil, lactamide MEA, laneth-16, laneth-10 acetate, lanolin, lanolin acid, lanolin alcohol, lanolin oil, lanolin wax, lavender (lavandula angustifolia) oil, lecithin, lemon (citrus medica limonum) oil, linoleic acid, linolenic acid, macadamia temifolia nut oil, maltitol, matricaria (chamomilla recutita) oil, methyl glucose sesquistearate, methylsilanol PCA, mineral oil, mink oil, mortierella oil, myristyl lactate, myristyl myristate, myristyl propionate, neopentyl glycol dicaprylate/dicaprate, octyldodecanol, octyldodecyl myristate, octyldodecyl stearoyl stearate, octyl hydroxystearate, octyl palmitate, octyl salicylate, octyl stearate, oleic acid, olive (olea europaea) oil, orange (citrus aurantium dulcis) oil, palm (elaeis guineensis) oil, palmitic acid, pantethine, panthenol, panthenyl ethyl ether, paraffin, PCA, peach (prunus persica) kernel oil, peanut (arachis hypogaea) oil, PEG-8 C12-18 ester, PEG-15 cocamine, PEG-150 distearate, PEG-60 glyceryl isostearate, PEG-5 glyceryl stearate, PEG-30 glyceryl stearate, PEG-7 hydrogenated castor oil, PEG-40 hydrogenated castor oil, PEG-60 hydrogenated castor oil, PEG-20 methyl glucose sesquistearate, PEG-40 sorbitan peroleate, PEG-5 soy sterol, PEG-10 soy sterol, PEG-2 stearate, PEG-8 stearate, PEG-20 stearate, PEG-32 stearate, PEG-40 stearate, PEG-50 stearate, PEG-100 stearate, PEG-150 stearate, pentadecalactone, peppermint (mentha piperita) oil, petrolatum, phospholipids, polyamino sugar condensate, polyglyceryl-3 diisostearate, polyquaternium-24, polysorbate 20, polysorbate 40, polysorbate 60, polysorbate 80, polysorbate 85, potassium myristate, potassium palmitate, propylene glycol, propylene glycol dicaprylate/dicaprate, propylene glycol dioctanoate, propylene glycol dipelargonate, propylene glycol laurate, propylene glycol stearate, propylene glycol stearate SE, PVP, pyridoxine dipalmitate, retinol, retinyl palmitate, rice (oryza sativa) bran oil, RNA, rosemary (rosmarinus officinalis) oil, rose oil, safflower (carthamus tinctorius) oil, sage (salvia officinalis) oil, sandalwood (santalum album) oil, serine, serum protein, sesame (sesamum indicum) oil, shea butter (butyrospermum parkii), silk powder, sodium chondroitin sulfate, sodium hyaluronate, sodium lactate, sodium palmitate, sodium PCA, sodium polyglutamate, soluble collagen, sorbitan laurate, sorbitan oleate, sorbitan palmitate, sorbitan sesquioleate, sorbitan stearate, sorbitol, soybean (glycine soja) oil, sphingolipids, squalane, squalene, stearamide MEA-stearate, stearic acid, stearoxy dimethicone, stearoxytrimethylsilane, stearyl alcohol, stearyl glycyrrhetinate, stearyl heptanoate, stearyl stearate, sunflower (helianthus annuus) seed oil, sweet almond (prunus amygdalus dulcis) oil, synthetic beeswax, tocopherol, tocopheryl acetate, tocopheryl linoleate, tribehenin, tridecyl neopentanoate, tridecyl stearate, triethanolamine, tristearin, urea, vegetable oil, water, waxes, wheat (triticum vulgare) germ oil, and ylang ylang (cananga odorata) oil.

### c. Antioxidants

Some examples of antioxidants that can be used with the compositions of the disclosure include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite.

### d. Structuring Agents

In other aspects, the compositions of the disclosure can include a structuring agent. Structuring agent, in certain aspects, assist in providing rheological characteristics to the composition to contribute to the composition's stability. In other aspects, structuring agents can also function as an emulsifier or surfactant. Some examples of structuring agents include stearic acid, palmitic acid, stearyl alcohol, cetyl alcohol, PPG-30 cetyl ether, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 5 ethylene oxide units, polyoxyethylene methylglucoside dioleate, tea-lauryl sulfate, polyethylene glycol ester of stearic acid, C₁₂₋₁₅ alkyl benzoate, propylene glycol myristyl ether acetate, 3-hydroxypropyl (E)-octadec-9-enoate, sorbitan laurate, sorbitan stearate, carbomer, ammonium acryloyldimethyltaurate/carboxyethyl acrylate crosspolymer, sodium laureth sulfate, hydroxypropyl cyclodextrin, PPG-26 oleate, and mixtures thereof.

### e. Emulsifiers

In certain aspects of the disclosure, the compositions do not include an emulsifier. In other aspects, however, the compositions can include one or more emulsifiers. Emulsifiers can reduce the interfacial tension between phases and improve the formulation and stability of an emulsion. The emulsifiers can be nonionic, cationic, anionic, and zwitterionic emulsifiers (See McCutcheon's (1986); U.S. Pat. Nos. 5,011,681; 4,421,769; 3,755,560). Some examples include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps, TEA stearate, DEA oleth-3 phosphate, polyethylene glycol 20 sorbitan monolaurate (polysorbate 20), polyethylene glycol 5 soya sterol, steareth-2, steareth-20, steareth-21, ceteareth-20, cetearyl glucoside, cetearyl alcohol, C12-13 pareth-3, PPG-2 methyl glucose ether distearate, PPG-5-ceteth-20, bis-PEG/PPG-20/20 dimethicone, ceteth-10, polysorbate 80, cetyl phosphate, potassium cetyl phosphate, diethanolamine cetyl phosphate, polysorbate 60, glyceryl stearate, PEG-100 stearate, C20-40 alcohols, polyhydroxystearic acid, and mixtures thereof.

### f. Silicone Containing Compounds

In some aspects, silicone containing compounds include any member of a family of polymeric products whose molecular backbone is made up of alternating silicon and oxygen atoms with side groups attached to the silicon atoms. By varying the -Si-O- chain lengths, side groups, and crosslinking, silicones can be synthesized into a wide variety of materials. They can vary in consistency from liquid to gel to solids.

The silicone containing compounds that can be used in the context of the compositions described herein include those described in this specification or those known to a person of ordinary skill in the art. Some examples include silicone oils *(e.g.,* volatile and non-volatile oils), gels, and solids. In certain aspects, the silicon containing compounds includes a silicone oils such as a polyorganosiloxane. Non-limiting examples of polyorganosiloxanes include dimethicone, cyclomethicone, polysilicone-11, phenyl trimethicone, trimethylsilylamodimethicone, stearoxytrimethylsilane, or mixtures of these and other organosiloxane materials in any given ratio in order to achieve the desired consistency and application characteristics depending upon the intended application (e.g., to a particular area such as the skin, hair, or eyes). A "volatile silicone oil" includes a silicone oil have a low heat of vaporization, e.g. normally less than about 50 cal per gram of silicone oil. Non-limiting examples of volatile silicone oils include: cyclomethicones such as Dow Corning 344 Fluid, Dow Corning 345 Fluid, Dow Corning 244 Fluid, and Dow Corning 245 Fluid, Volatile Silicon 7207 (Union Carbide Corp., Danbury, Conn.); low viscosity dimethicones, *e.g.* dimethicones having a viscosity of about 50 cst or less (*e.g.,* dimethicones such as Dow Corning 200-0.5 cst Fluid). The Dow Corning Fluids are available from Dow Corning Corporation, Midland, Michigan. Cyclomethicone and dimethicone are described in the Third Edition of the CTFA Cosmetic Ingredient Dictionary (incorporated by reference) as cyclic dimethyl polysiloxane compounds and a mixture of fully methylated linear siloxane polymers end-blocked with trimethylsiloxy units, respectively. Silicone containing compounds may also be used as bulking agents (*e.g*. silicic acid, silica, synthetic fluorphlogopite, calcium aluminum borosilicate, calcium sodium borosilicate, and aluminum calcium sodium silicate). Other non-limiting volatile silicone oils that can be used include those available from General Electric Co., Silicone Products Div., Waterford, N.Y. and SWS Silicones Div. of Stauffer Chemical Co., Adrian, Michigan.

### g. Essential Oils

Essential oils include oils derived from herbs, flowers, trees, and other plants. Such oils are typically present as tiny droplets between the plant's cells, and can be extracted by several methods known to those of skill in the art (*e.g.,* steam distilled, enfleurage (*e.g.,* extraction by using fat), maceration, solvent extraction, or mechanical pressing). When these types of oils are exposed to air they tend to evaporate (*e.g*., a volatile oil). As a result, many essential oils are colorless, but with age they can oxidize and become darker. Essential oils are insoluble in water and are soluble in alcohol, ether, fixed oils (vegetal), and other organic solvents. Typical physical characteristics found in essential oils include boiling points that vary from about 160° to 240° C and densities ranging from about 0.759 to about 1.096.

Essential oils typically are named by the plant from which the oil is found. For example, rose oil or peppermint oil are derived from rose or peppermint plants, respectively. Non-limiting examples of essential oils that can be used include sesame oil, macadamia nut oil, tea tree oil, evening primrose oil, Spanish sage oil, Spanish rosemary oil, coriander oil, thyme oil, pimento berries oil, rose oil, anise oil, balsam oil, bergamot oil, rosewood oil, cedar oil, chamomile oil, sage oil, clary sage oil, clove oil, cypress oil, eucalyptus oil, fennel oil, sea fennel oil, frankincense oil, geranium oil, ginger oil, grapefruit oil, jasmine oil, juniper oil, lavender oil, lemon oil, lemongrass oil, lime oil, mandarin oil, marjoram oil, myrrh oil, neroli oil, orange oil, patchouli oil, pepper oil, black pepper oil, petitgrain oil, pine oil, rose otto oil, rosemary oil, sandalwood oil, spearmint oil, spikenard oil, vetiver oil, wintergreen oil, or ylang ylang. Other essential oils known to those of skill in the art are also contemplated as being useful in the compositions described herein.

### h. Thickening Agents

Thickening agents, including thickener or gelling agents, include substances which that can increase or control the viscosity of a composition. Thickeners includes those that can increase the viscosity of a composition without substantially modifying the efficacy of the active ingredient within the composition. Thickeners can also increase the stability of the compositions of the disclosure. In certain aspects, thickeners include hydrogenated polyisobutene or trihydroxystearin, or a mixture thereof.

Some examples of additional thickening agents that can be used include carboxylic acid polymers, crosslinked polyacrylate polymers, polyacrylamide polymers, polysaccharides, and gums. Examples of carboxylic acid polymers include crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol (see U.S. Pat. Nos. 5,087,445; 4,509,949; 2,798,053; CTFA International Cosmetic Ingredient Dictionary, Fourth edition, 1991, pp. 12 and 80). Examples of commercially available carboxylic acid polymers include carbomers, which are homopolymers of acrylic acid crosslinked with allyl ethers of sucrose or pentaerytritol (*e.g*., Carbopol™ 900 series from B. F. Goodrich).

Some examples of crosslinked polyacrylate polymers include cationic and nonionic polymers. Examples are described in U.S. Pat. Nos. 5,100,660 ; 4,849,484; 4,835,206; 4,628,078; 4,599,379.

Some examples of polyacrylamide polymers (including nonionic polyacrylamide polymers including substituted branched or unbranched polymers) include polyacrylamide, isoparaffin and laureth-7, multi-block copolymers of acrylamides and substituted acrylamides with acrylic acids and substituted acrylic acids.

Some examples of polysaccharides include cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Another example is an alkyl substituted cellulose where the hydroxy groups of the cellulose polymer is hydroxyalkylated (preferably hydroxy ethylated or hydroxypropylated) to form a hydroxyalkylated cellulose which is then further modified with a C₁₀ -C₃₀ straight chain or branched chain alkyl group through an ether linkage. Typically these polymers are ethers of C₁₀-C₃₀ straight or branched chain alcohols with hydroxyalkylcelluloses. Other useful polysaccharides include scleroglucans comprising a linear chain of (1-3) linked glucose units with a (1-6) linked glucose every three unit.

Some examples of gums that can be used include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

Further examples of thickening agents include carbomer, cetyl alcohol, ammonium acryloydimethyltaurate/VP copolymer, aluminum starch actenylsuccinate, cocamidopropyl betaine, PPG-2 hydroxyethyl coco/isostearamide, tin oxide, hexadecane copolymer, calcium aluminum borosilicate, alumina, calcium sodium borosilicate, aluminum calcium sodium silicate, synthetic fluorphlogopite, ozokerite, microcrystalline wax, stearalkonium hectorite, propylene carbonate, and disodium EDTA.

### i. Preservatives

Some examples of preservatives that can be used include quaternary ammonium preservatives such as polyquaternium-1 and benzalkonium halides (*e.g.,* benzalkonium chloride ("BAC") and benzalkonium bromide), parabens (*e.g*., methylparabens and propylparabens), phenoxyethanol, benzyl alcohol, chlorobutanol, phenol, sorbic acid, thimerosal, caprylyl glycol, iodopropynyl butylcarbamate, methylisothiazolinone, methylchloroisothiazolinone, sodium benzoate, dimethylol-5,5-dimethylhydantoin, 3-iodo-2-propynyl butyl carbamate, phenoxyethanol, caprylyl alcohol, ethylhexyl glycerin, hexylene glycol, DMDM hydantoin, chlorphenesin, or combinations thereof.

### j. Conditioning Agents

Some examples of conditioning agents that can be used include caprylyl glycol, ethylhexylglycerin, PEG-12 dimethicone, hydroxypropyl cyclodextrin, dimethicone, tocopheryl acetate, *Butyrospermum parkii* (shea butter), polymers of polyethylene glycol and methicone, *Helianthus annuus* (sunflower) seed oil, PEG-18 glyceryl oleate/cocoate, cyclotetrasiloxane, cyclohexasiloxane, cyclopentasiloxane, tocopherol, glycerin, *Carthamus tinctorius* (safflower) oleosomes, *Opuntia tuna* fruit extract, butylene glycol, allantoin, hydrogenated palm kernel oil, caprylic/capric triglyceride, propylene glycol stearate, panthenol, polypropylene glycol ether of cetyl alcohol, polyquaternium-7, ethoxylated glyceryl esters, ethylhexyl palmitate. aloe extracts, bisabolol, ceramides, hyaluronic acid, dipotassium glycyrrhizate, cocamidopropyl betaine, pentaerythrityl tetraisostearate, glyceryl behenate/eicosadioate, tridecyl trimellitate, sucrose polysoyate, bis-diglyceryl polyacyladipate-2, butyl stearate, isodecyl neopentanoate, isononyl isononanoate, and mixtures thereof.

### E. Kits

Kits are also contemplated as being used in certain aspects of the disclosure but are not part of the invention as claimed. For instance, compositions described herein can be included in a kit. A kit can include a container. Containers can include a bottle, a metal tube, a laminate tube, a plastic tube, a dispenser, a pressurized container, a barrier container, a package, a compartment, a lipstick container, a compact container, cosmetic pans that can hold cosmetic compositions, or other types of containers such as injection or blow-molded plastic containers into which the dispersions or compositions or desired bottles, dispensers, or packages are retained. The kit and/or container can include indicia on its surface. The indicia, for example, can be a word, a phrase, an abbreviation, a picture, or a symbol.

The containers can dispense a pre-determined amount of the composition. In other embodiments, the container can be squeezed (*e.g*., metal, laminate, or plastic tube) to dispense a desired amount of the composition. The composition can be dispensed as a spray, an aerosol, a liquid, a fluid, or a semi-solid. The containers can have spray, pump, or squeeze mechanisms. A kit can also include instructions for employing the kit components as well the use of any other compositions included in the container. Instructions can include an explanation of how to apply, use, and maintain the compositions.

### EXAMPLES

The following examples are included to demonstrate certain aspects of the disclosure.

### EXAMPLE 1

### Lipstick Formulations

The compositions listed in Tables 1-18 are compositions that can be used in the context of aspects of the present invention. Only the composition of table 3 illustrates the invention as claimed. Compositions of tables 1-2, 4-18 are not part of the invention as claimed.

**Table 1***

| **Ingredient**** | **% Concentration (by weight)** |
|---|---|
| Stearalkonium Hectorite | 0.06 |
| Polyhydroxystearic Acid | 0.02 |
| Propylene Carbonate | 0.02 |
| *Helianthus annus* (sunflower) Seed Oil | 6 |
| Pentaerythrityl Tetraisostearate | 11 |
| Bis-diglyceryl Polyacyladipate-2 | 10 |
| Isononyl Isononanoate | 10 |
| Polyethylene | 6 |
| *Simmondsia chinensis* (jojoba) Butter | 5 |
| Mica | 5 |
| Ozokerite | 3 |
| Sucrose Polysoyate | 3 |
| Silica | 2 |
| Microcrystalline Wax/Cire Microcrystalline | 2 |
| HDI/Trimethylol Hexyllactone Crosspolymer | 2 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 2***

| **Ingredient**** | **% Concentration (by weight)** |
|---|---|
| Stearalkonium Hectorite | 0.06 |
| Polyhydroxystearic Acid | 0.02 |
| Propylene Carbonate | 0.02 |
| *Helianthus annus* (sunflower) Seed Oil | 6 |
| Pentaerythrityl Tetraisostearate | 11 |
| Bis-diglyceryl Polyacyladipate-2 | 10 |
| Isononyl Isononanoate | 10 |
| Triisostearyl Citrate | 10 |
| Polyethylene | 6 |
| *Simmondsia chinensis* (jojoba) Butter | 5 |
| Mica | 5 |
| Ozokerite | 3 |
| Sucrose Polysoyate | 3 |
| Silica | 2 |
| Microcrystalline Wax/Cire Microcrystalline | 2 |
| HDI/Trimethylol Hexyllactone Crosspolymer | 2 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 3***

| **Ingredient**** | **% Concentration (by weight)** |
|---|---|
| Stearalkonium Hectorite | 0.04 |
| Polyhydroxystearic Acid | 0.005 |
| Propylene Carbonate | 0.01 |
| *Helianthus annus* (sunflower) Seed Oil | 12 |
| Pentaerythrityl Tetraisostearate | 11 |
| Bis-diglyceryl Polyacyladipate-2 | 11 |
| Isononyl Isononanoate | 10 |
| Polyethylene | 5 |
| *Simmondsia chinensis* (jojoba) Butter | 6 |
| Mica | 9 |
| Ozokerite | 2 |
| Sucrose Polysoyate | 5 |
| Silica | 4 |
| Microcrystalline Wax/Cire Microcrystalline | 0.5 |
| HDI/Trimethylol Hexyllactone Crosspolymer | 4 |
| Polyglyceryl-10 Pentaisostearate | 6 |
| Glyceryl Behenate/Eicosadioate | 0.5 |
| Isopropyl Myristate | 0.5 |
| Tocopheryl Acetate | 0.5 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 4***

| **Ingredient**** | **% Concentration (by weight)** |
|---|---|
| Stearalkonium Hectorite | 0.06 |
| Polyhydroxystearic Acid | 0.04 |
| Propylene Carbonate | 0.02 |
| *Helianthus annus* (sunflower) Seed Oil | 13 |
| Pentaerythrityl Tetraisostearate | 12 |
| Bis-diglyceryl Polyacyladipate-2 | 12 |
| Isononyl Isononanoate | 10 |
| Polyethylene | 5 |
| *Simmondsia chinensis* (jojoba) Butter | 6 |
| Mica | 11 |
| Ozokerite | 2 |
| Sucrose Polysoyate | 5 |
| Silica | 5 |
| Microcrystalline Wax/Cire Microcrystalline | 0.6 |
| Mixture of HDI/Trimethylol Hexyllactone Crosspolymer and Methylsilsesquioxane | 12 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 5***

| **Ingredient**** | **% Concentration (by weight)** |
|---|---|
| Stearalkonium Hectorite | 0.05 |
| Polyhydroxystearic Acid | 0.02 |
| Propylene Carbonate | 0.02 |
| *Helianthus annus* (sunflower) Seed Oil | 6 |
| Pentaerythrityl Tetraisostearate | 4 |
| Bis-diglyceryl Polyacyladipate-2 | 11 |
| Isononyl Isononanoate | 9 |
| Polyethylene | 5 |
| *Simmondsia chinensis* (jojoba) Butter | 5 |
| Mica | 5 |
| Ozokerite | 3 |
| Sucrose Polysoyate | 3 |
| Silica | 2 |
| Microcrystalline Wax/Cire Microcrystalline | 2 |
| HDI/Trimethylol Hexyllactone Crosspolymer | 2 |
| Polyglyceryl-2 Diisostearate/IPDI Copolymer | 12 |
| Triisostearyl Citrate | 10 |
| DI-PPG-3 Myristyl ether Adipate | 6 |
| Hydrogenated Polyisobutene | 4 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 6***

| **Ingredient**** | **% Concentration (by weight)** |
|---|---|
| *Ricinus communis* Seed Oil | 24 |
| Oleyl Oleate | 10 |
| Beeswax | 5 |
| *Euphorbia cerifera* Wax | 5 |
| Ozokerite | 5 |
| *Copernicia cerifera* Wax | 2 |
| Methyl Glucose Sesquistearate | 2 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 7***

| **Ingredient**** | **% Concentration (by weight)** |
|---|---|
| *Ricinus communis* Seed Oil | 48 |
| Oleyl Oleate | 10 |
| Beeswax | 7 |
| *Euphorbia cerifera* Wax | 6 |
| Ozokerite | 6 |
| *Copernicia cerifera* Wax | 2 |
| Methyl Glucose Sesquistearate | 2 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 8***

| **Ingredient**** | **% Concentration (by weight)** |
|---|---|
| *Ricinus communis* Seed Oil | 50 |
| Oleyl Oleate | 10 |
| Beeswax | 5 |
| *Euphorbia cerifera* Wax | 5 |
| Ozokerite | 5 |
| *Copernicia cerifera* Wax | 2 |
| Methyl Glucose Sesquistearate | 2 |
| Colorant(s) | 10.5 |
| Titanium Dioxide | 0.5 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 9***

| **Ingredient**** | **% Concentration (by weight)** |
|---|---|
| *Helianthus annuus* Oil | 13 |
| Pentaerythrityl Tetraisostearate | 12 |
| Mixture of HDI/Trimethylol Hexyllactone Crosspolymer and Silica | 12 |
| Mica | 11 |
| Bis-diglyceryl Polyacyladipate-2 | 9 |
| *Simmondsia chinesis* Butter | 6 |
| Polyethylene | 6 |
| Ozokerite | 3 |
| Microcrystalline Wax | 0.6 |
| Glyceryl Behenate/Eicosadioate | 0.5 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 10***

| **Ingredient**** | **% Concentration (by weight)** |
|---|---|
| Hybrid *Helianthus annuus* Oil | 13 |
| Pentaerythrityl Tetraisostearate | 12 |
| Mixture of HDI/Trimethylol Hexyllactone Crosspolymer and Silica | 16 |
| Mica | 10 |
| Bis-diglyceryl Polyacyladipate-2 | 9 |
| *Simmondsia chinesis* Butter | 6 |
| Polyethylene | 5 |
| Ozokerite | 2 |
| Microcrystalline Wax | 0.7 |
| Glyceryl Behenate/Eicosadioate | 0.5 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 11***

| **Ingredient**** | **% Concentration (by weight)** |
|---|---|
| *Helianthus annuus* Oil | 12 |
| Pentaerythrityl Tetraisostearate | 14 |
| Mixture of HDI/Trimethylol Hexyllactone Crosspolymer and Silica | 14 |
| Mica | 10 |
| Bis-diglyceryl Polyacyladipate-2 | 10 |
| *Simmondsia chinesis* Butter | 5 |
| Polyethylene | 5 |
| Ozokerite | 2 |
| Microcrystalline Wax | 0.7 |
| Glyceryl Behenate/Eicosadioate | 0.5 |
| Colorant(s) | 3.2 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 12***

| **Ingredient**** | **% Concentration (by weight)** |
|---|---|
| Ethylhexyl Palmitate | 1 |
| Tribehenin | 0.2 |
| Sorbitan Isostearate | 0.05 |
| Palmitoyl Oligopeptide | 0.001 |
| *Ricinus communis* (castor) Seed Oil | 33 |
| Jojoba Esters | 13 |
| *Helianthus annus* (sunflower) Seed Oil | 12 |
| Ethyl Macadamiate | 6 |
| *Euphorbia cerifera* (candelilla) Wax | 6 |
| *Mangifera indica* (mango) Seed Butter | 5 |
| Mica | 5 |
| *Limnanthes alba* (meadowfoam) Seed Oil | 4 |
| Beeswax | 4 |
| *Helianthus annus* (sunflower) Seed Wax | 4 |
| Sucrose Acetate Isobutyrate | 3 |
| *Citrus aurantium dulcis* (orange) Peel Wax | 1 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 13***

| **Ingredient**** | **% Concentration (by weight)** |
|---|---|
| *Helianthus annuus* (Sunflower) Seed Oil | 13 |
| Pentaerythrityl Tetraisostearate | 12 |
| Bis-diglyceryl Polyacyladipate-2 | 12 |
| Mixture of HDI/Trimethylol Hexyllactone Crosspolymer and Methylsilsesquioxane | 12 |
| Mica | 11 |
| Isononyl Isononanoate | 10 |
| *Simmondsia chinesis* (Jojoba) Butter | 6 |
| Polyethylene | 5 |
| Silica | 5 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 14***

| **Ingredient**** | **% Concentration (by weight)** |
|---|---|
| *Helianthus annuus* (Sunflower) Seed Oil | 13 |
| Pentaerythrityl Tetraisostearate | 12 |
| Bis-diglyceryl Polyacyladipate-2 | 12 |
| Mixture of HDI/Trimethylol Hexyllactone Crosspolymer and Methylsilsesquioxane | 12 |
| Mica | 11 |
| Isononyl Isononanoate | 10 |
| *Simmondsia chinesis* (Jojoba) Butter | 6 |
| Polyethylene | 5 |
| Silica | 5 |
| Colorant(s) | 5 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 15***

| **Ingredient**** | **% Concentration (by weight)** |
|---|---|
| *Helianthus annuus* (Sunflower) Seed Oil | 13 |
| Pentaerythrityl Tetraisostearate | 12 |
| Bis-diglyceryl Polyacyladipate-2 | 12 |
| Mixture of HDI/Trimethylol Hexyllactone Crosspolymer and Methylsilsesquioxane | 12 |
| Mica | 11 |
| Isononyl Isononanoate | 10 |
| *Simmondsia chinesis* (Jojoba) Butter | 6 |
| Polyethylene | 5 |
| Silica | 5 |
| Iron Oxides | 3 |
| Blue 1 Lake | 0.5 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 16***

| **Ingredient**** | **% Concentration (by weight)** |
|---|---|
| *Helianthus annuus* (Sunflower) Seed Oil | 13 |
| Pentaerythrityl Tetraisostearate | 12 |
| Bis-diglyceryl Polyacyladipate-2 | 12 |
| Mixture of HDI/Trimethylol Hexyllactone Crosspolymer and Methylsilsesquioxane | 12 |
| Mica | 11 |
| Isononyl Isononanoate | 10 |
| *Simmondsia chinesis* (Jojoba) Butter | 6 |
| Polyethylene | 5 |
| Silica | 5 |
| Colorant(s) | 2 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 17***

| **Ingredient**** | **% Concentration (by weight)** |
|---|---|
| Polyglyceryl-2 Diisostearate/IPDI Copolymer | 12 |
| Bis-diglyceryl Polyacrladipate-2 | 11 |
| Triisostearyl Citrate | 10 |
| Isononyl Isononanoate | 9 |
| Polyethylene | 6 |
| DI-PPG-3 Myristyl Ether Adipate | 6 |
| *Helianthus annus* Seed Oil | 6 |
| *Simmondsia chinensis* Butter | 5 |
| Mica | 5 |
| Hydrogenated Polyisobutene | 4 |
| Pentaerythrityl Tetraisostearate | 4 |
| Ozokerite | 3 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

**Table 18***

| **Ingredient**** | **% Concentration (by weight)** |
|---|---|
| Polyglyceryl-2 Diisostearate/IPDI Copolymer | 12 |
| Bis-diglyceryl Polyacrladipate-2 | 11 |
| Triisostearyl Citrate | 10 |
| Isononyl Isononanoate | 10 |
| Polyethylene | 6 |
| DI-PPG-3 Myristyl Ether Adipate | 6 |
| *Helianthus annus* Seed Oil | 6 |
| *Simmondsia chinensis* Butter | 6 |
| Mica | 5 |
| Hydrogenated Polyisobutene | 4 |
| Pentaerythrityl Tetraisostearate | 4 |
| Ozokerite | 3 |
| Colorant(s) | 4 |
| Excipients*** | q.s. |

| | |
|---|---|
| *Formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition. **Any of the additional ingredients (or combination thereof) described in the specification can be used. ***Excipients can be added, for example, to modify the rheological properties of the composition. | |

### EXAMPLE 2

### Assays

The efficacy of the combination of ingredients disclosed throughout the specification and claims can be determined by using the following assays.
**Skin Moisture/Hydration Assay:** Skin moisture/hydration benefits can be measured by using impedance measurements with the Nova Dermal Phase Meter. The impedance meter measures changes in skin moisture content. The outer layer of the skin has distinct electrical properties. When skin is dry it conducts electricity very poorly. As it becomes more hydrated increasing conductivity results. Consequently, changes in skin impedance (related to conductivity) can be used to assess changes in skin hydration. The unit can be calibrated according to instrument instructions for each testing day. A notation of temperature and relative humidity can also be made. Subjects can be evaluated as follows: prior to measurement they can equilibrate in a room with defined humidity (*e.g*., 30-50%) and temperature (*e.g*., 68-72°C). Three separate impedance readings can be taken on each side of the face, recorded, and averaged. The T5 setting can be used on the impedance meter which averages the impedance values of every five seconds application to the face. Changes can be reported with statistical variance and significance.
**Skin Dryness, Surface Fine Lines, Skin Smoothness, and Skin Tone Assay:** Skin dryness, surface fine lines, skin smoothness, and skin tone can be evaluated with clinical grading techniques. For example, clinical grading of skin dryness can be determined by a five point standard Kligman Scale: (0) skin is soft and moist; (1) skin appears normal with no visible dryness; (2) skin feels slightly dry to the touch with no visible flaking; (3) skin feels dry, tough, and has a whitish appearance with some scaling; and (4) skin feels very dry, rough, and has a whitish appearance with scaling. Evaluations can be made independently by two clinicians and averaged.
**Clinical Grading of Skin Tone Assay:** Clinical grading of skin tone can be performed *via* a ten point analog numerical scale: (10) even skin of uniform, pinkish brown color. No dark, erythremic, or scaly patches upon examination with a hand held magnifying lens. Microtexture of the skin very uniform upon touch; (7) even skin tone observed without magnification. No scaly areas, but slight discolorations either due to pigmentation or erythema. No discolorations more than 1 cm in diameter; (4) both skin discoloration and uneven texture easily noticeable. Slight scaliness. Skin rough to the touch in some areas; and (1) uneven skin coloration and texture. Numerous areas of scaliness and discoloration, either hypopigmented, erythremic or dark spots. Large areas of uneven color more than 1 cm in diameter. Evaluations were made independently by two clinicians and averaged.
**Clinical Grading of Skin Smoothness Assay:** Clinical grading of skin smoothness can be analyzed *via* a ten point analog numerical scale: (10) smooth, skin is moist and glistening, no resistance upon dragging finger across surface; (7) somewhat smooth, slight resistance; (4) rough, visibly altered, friction upon rubbing; and (1) rough, flaky, uneven surface. Evaluations were made independently by two clinicians and averaged.
**Skin Smoothness and Wrinkle Reduction Assay With Methods Disclosed in Packman *et al.* (1978):** Skin smoothness and wrinkle reduction can also be assessed visually by using the methods disclosed in Packman *et al.* (1978). For example, at each subject visit, the depth, shallowness and the total number of superficial facial lines (SFLs) of each subject can be carefully scored and recorded. A numerical score was obtained by multiplying a number factor times a depth/width/length factor. Scores are obtained for the eye area and mouth area (left and right sides) and added together as the total wrinkle score.
**Skin Firmness Assay with a Hargens Ballistometer:** Skin firmness can be measured using a Hargens ballistometer, a device that evaluates the elasticity and firmness of the skin by dropping a small body onto the skin and recording its first two rebound peaks. The ballistometry is a small lightweight probe with a relatively blunt tip (4 square mm-contact area) was used. The probe penetrates slightly into the skin and results in measurements that are dependent upon the properties of the outer layers of the skin, including the stratum corneum and outer epidermis and some of the dermal layers.
**Skin Softness/Suppleness Assay with a Gas Bearing Electrodynamometer:** Skin softness/suppleness can be evaluated using the Gas Bearing Electrodynamometer, an instrument that measures the stress/strain properties of the skin. The viscoelastic properties of skin correlate with skin moisturization. Measurements can be obtained on the predetermined site on the cheek area by attaching the probe to the skin surface with double-stick tape. A force of approximately 3.5 gm can be applied parallel to the skin surface and the skin displacement is accurately measured. Skin suppleness can then be calculated and is expressed as DSR (Dynamic Spring Rate in gm/mm).
**Appearance of Lines and Wrinkles Assay with Replicas:** The appearance of lines and wrinkles on the skin can be evaluated using replicas, which is the impression of the skin's surface. Silicone rubber like material can be used. The replica can be analyzed by image analysis. Changes in the visibility of lines and wrinkles can be objectively quantified *via* the taking of silicon replicas form the subjects' face and analyzing the replicas image using a computer image analysis system. Replicas can be taken from the eye area and the neck area, and photographed with a digital camera using a low angle incidence lighting. The digital images can be analyzed with an image processing program and the area of the replicas covered by wrinkles or fine lines was determined.
**Surface Contour of the Skin Assay with a Profilometer/Stylus Method:** The surface contour of the skin can be measured by using the profilometer/Stylus method. This includes either shining a light or dragging a stylus across the replica surface. The vertical displacement of the stylus can be fed into a computer *via* a distance transducer, and after scanning a fixed length of replica a cross-sectional analysis of skin profile can be generated as a two-dimensional curve. This scan can be repeated any number of times along a fix axis to generate a simulated 3-D picture of the skin. Ten random sections of the replicas using the stylus technique can be obtained and combined to generate average values. The values of interest include Ra which is the arithmetic mean of all roughness (height) values computed by integrating the profile height relative to the mean profile height. Rt which is the maximum vertical distance between the highest peak and lowest trough, and Rz which is the mean peak amplitude minus the mean peak height. Values are given as a calibrated value in mm. Equipment should be standardized prior to each use by scanning metal standards of know values. Ra Value can be computed by the following equation: Rₐ = Standardize roughness; *l*ₘ = the traverse (scan) length; and y = the absolute value of the location of the profile relative to the mean profile height (x-axis).
**MELANODERM™ Assay:** In other non-limiting aspects, the efficacy of the compositions can be evaluated by using a skin analog, such as, for example, MELANODERM™. Melanocytes, one of the cells in the skin analog, stain positively when exposed to L-dihydroxyphenyl alanine (L-DOPA), a precursor of melanin. The skin analog, MELANODERM™, can be treated with a variety of bases containing the compositions and whitening agents of the present disclosure or with the base alone as a control. Alternatively, an untreated sample of the skin analog can be used as a control.
**ORAC Assay:** Oxygen Radical Absorption (or Absorbance) Capacity (ORAC) of the aromatic skin-active ingredients and compositions can also be assayed by measuring the antioxidant activity of such ingredients or compositions. This assay can quantify the degree and length of time it takes to inhibit the action of an oxidizing agent such as oxygen radicals that are known to cause damage cells (*e.g.,* skin cells). The ORAC value of the aromatic skin-active ingredients and compositions can be determined by methods known to those of ordinary skill in the art (*see* U.S. Publication Nos. 2004/0109905 and 2005/0163880; Cao *et al.* (1993)), all of which are incorporated by reference). In summary, the assay described in Cao *et al.* (1993) measures the ability of antioxidant compounds in test materials to inhibit the decline of B-phycoerythrm (B-PE) fluorescence that is induced by a peroxyl radical generator, AAPH.
**Matrix Metalloproteinase Enzyme Activity (MMP3; MMP9) Assay:** An *in vitro* matrix metalloprotease (MMP) inhibition assay. MMPs are extracellular proteases that play a role in many normal and disease states by virtue of their broad substrate specificity. MMP3 substrates include collagens, fibronectins, and laminin; while MMP9 substrates include collagen VII, fibronectins and laminin. Using Colorimetric Drug Discovery kits from BioMol International for MMP3 (AK-400) and MMP-9 (AK-410), this assay is designed to measure protease activity of MMPs using a thiopeptide as a chromogenic substrate (Ac-PLG-[2-mercapto-4-methyl-pentanoyl]-LG-OC2H5)5,6. The MMP cleavage site peptide bond is replaced by a thioester bond in the thiopeptide. Hydrolysis of this bond by an MMP produces a sulfhydryl group, which reacts with DTNB [5,5'-dithiobis(2-nitrobenzoic acid), Ellman's reagent] to form 2-nitro-5- thiobenzoic acid, which can be detected by its absorbance at 412 nm (ε=13,600 M-1cm-1 at pH 6.0 and above 7).
**Collagen Stimulation Assay:** Collagen is an extracellular matrix protein critical for skin structure. Increased synthesis of collagen helps improve skin firmness and elasticity. This bioassay can be used to examine the effect of any one of the active ingredients, combination of ingredients, or compositions having said combinations disclosed in the specification on the production of procollagen peptide (a precursor to collagen) by human epidermal fibroblasts. The endpoint of this assay is a spectrophotometric measurement that reflects the presence of procollagen peptide and cellular viability. The assay employs the quantitative sandwich enzyme immunoassay technique whereby a monoclonal antibody specific for procollagen peptide has been pre-coated onto a microplate. Standards and samples can be pipetted into the wells and any procollagen peptide present is bound by the immobilized antibody. After washing away any unbound substances, an enzyme-linked polyclonal antibody specific for procollagen peptide can be added to the wells. Following a wash to remove any unbound antibody-enzyme reagent, a substrate solution can be added to the wells and color develops in proportion to the amount of procollagen peptide bound in the initial step using a microplate reader for detection at 450 nm. The color development can be stopped and the intensity of the color can be measured. Subconfluent normal human adult epidermal fibroblasts (Cascade Biologics) cultivated in standard DMEM growth medium with 10% fetal bovine serum (Mediatech) at 37°C in 10% CO₂, can be treated with each of the combination of ingredients or compositions having said combinations disclosed in the specification for 3 days. Following incubation, cell culture medium can be collected and the amount of procollagen peptide secretion quantified using a sandwich enzyme linked immuno-sorbant assay (ELISA) from Takara (#MK101).
**Tumor Necrosis Factor Alpha (TNF-α) Assay:** The prototype ligand of the TNF superfamily, TNF-α, is a pleiotropic cytokine that plays a central role in inflammation. Increase in its expression is associated with an up regulation in pro-inflammatory activity. This bioassay can be used to analyze the effect of any one of the active ingredients, combination of ingredients, or compositions having said combinations disclosed in the specification on the production of TNF-α by human epidermal keratinocytes. The endpoint of this assay can be a spectrophotometric measurement that reflects the presence of TNF-α and cellular viability. The assay employs the quantitative sandwich enzyme immunoassay technique whereby a monoclonal antibody specific for TNF-α has been pre-coated onto a microplate. Standards and samples can be pipetted into the wells and any TNF-α present is bound by the immobilized antibody. After washing away any unbound substances, an enzyme-linked polyclonal antibody specific for TNF-α can be added to the wells. Following a wash to remove any unbound antibody-enzyme reagent, a substrate solution can be added to the wells and color develops in proportion to the amount of TNF-α bound in the initial step using a microplate reader for detection at 450 nm. The color development can be stopped and the intensity of the color can be measured. Subconfluent normal human adult keratinocytes (Cascade Biologics) cultivated in EpiLife standard growth medium (Cascade Biologics) at 37°C in 5% CO₂, can be treated with phorbol 12-myristate 13-acetate (PMA , 10ng/ml, Sigma Chemical, #P1585-1MG) and any one of the active ingredients, combination of ingredients, or compositions having said combinations disclosed in the specification for 6 hours. PMA has been shown to cause a dramatic increase in TNF-α secretion which peaks at 6 hours after treatment. Following incubation, cell culture medium can be collected and the amount of TNF-a secretion quantified using a sandwich enzyme linked immuno-sorbant assay (ELISA) from R&D Systems (#DTA00C).
**Antioxidant (AO) assay:** An *in vitro* bioassay that measures the total anti-oxidant capacity of any one of the ingredients, combination of ingredients, or compositions having said combinations disclosed in the specification. The assay relies on the ability of antioxidants in the sample to inhibit the oxidation of ABTS® (2,2'-azino-di-[3-ethylbenzthiazoline sulphonate]) to ABTS®+ by metmyoglobin. The antioxidant system of living organisms includes enzymes such as superoxide dismutase, catalase, and glutathione peroxidase; macromolecules such as albumin, ceruloplasmin, and ferritin; and an array of small molecules, including ascorbic acid, α-tocopherol, β-carotene, reduced glutathione, uric acid, and bilirubin. The sum of endogenous and food-derived antioxidants represents the total antioxidant activity of the extracellular fluid. Cooperation of all the different antioxidants provides greater protection against attack by reactive oxygen or nitrogen radicals, than any single compound alone. Thus, the overall antioxidant capacity may give more relevant biological information compared to that obtained by the measurement of individual components, as it considers the cumulative effect of all antioxidants present in plasma and body fluids. The capacity of the antioxidants in the sample to prevent ABTS oxidation is compared with that of Trolox, a water-soluble tocopherol analogue, and is quantified as molar Trolox equivalents. Anti-Oxidant capacity kit # 709001 from Cayman Chemical (Ann Arbor, Michigan USA) can be used as an *in vitro* bioassay to measure the total anti-oxidant capacity of each of any one of the active ingredients, combination of ingredients, or compositions having said combinations disclosed in the specification. The protocol can be followed according to manufacturer recommendations. The assay relied on antioxidants in the sample to inhibit the oxidation of ABTS® (2,2'-azino-di-[3-ethylbenzthiazoline sulphonate]) to ABTS®+ by metmyoglobin. The capacity of the antioxidants in the sample to prevent ABTS oxidation can be compared with that Trolox, a water-soluble tocopherol analogue, and was quantified as a molar Trolox equivalent.
**Mushroom tyrosinase activity assay:** In mammalian cells, tyrosinase catalyzes two steps in the multi-step biosynthesis of melanin pigments from tyrosine (and from the polymerization of dopachrome). Tyrosinase is localized in melanocytes and produces melanin (aromatic quinone compounds) that imparts color to skin, hair, and eyes. Purified mushroom tyrosinase (Sigma) can be incubated with its substrate L-Dopa (Fisher) in the presence or absence of each of the active ingredients, any one of the combination of ingredients, or compositions having said combinations disclosed in the specification. Pigment formation can be evaluated by colorimetric plate reading at 490 nm. The percent inhibition of mushroom tyrosinase activity can be calculated compared to non-treated controls to determine the ability of test ingredients or combinations thereof to inhibit the activity of purified enzyme. Test ingredient inhibition can be compared with that of kojic acid (Sigma).
**Cyclooxygenase (COX) Assay:** An *in vitro* cyclooxygenase-1 and -2 (COX-1, -2) inhibition assay. COX is a bifunctional enzyme exhibiting both cyclooxygenase and peroxidase activities. The cyclooxygenase activity converts arachidonic acid to a hydroperoxy endoperoxide (Prostaglandin G2; PGG2) and the peroxidase component reduces the endoperoxide (Prostaglandin H2; PGH2) to the corresponding alcohol, the precursor of prostaglandins, thromboxanes, and prostacyclins. This COX Inhibitor screening assay measures the peroxidase component of cyclooxygenases. The peroxidase activity is assayed colorimetrically by monitoring the appearance of oxidized N,N,N',N'-tetramethyl-p-phenylenediamine (TMPD). This inhibitor screening assay includes both COX-1 and COX-2 enzymes in order to screen isozyme-specific inhibitors. The Colormetric COX (ovine) Inhibitor screening assay (#760111, Cayman Chemical) can be used to analyze the effects of each of the active ingredients, any one of the combination of ingredients, or compositions having said combinations disclosed in the specification on the activity of purified cyclooxygnase enzyme (COX-1 or COX-2). According to manufacturer instructions, purified enzyme, heme and test ingredients can be mixed in assay buffer and incubated with shaking for 15 min at room temperature. Following incubation, arachidonic acid and colorimetric substrate can be added to initiate the reaction. Color progression can be evaluated by colorimetric plate reading at 590nm. The percent inhibition of COX-1 or COX-2 activity can be calculated compared to non-treated controls to determine the ability of test ingredients to inhibit the activity of purified enzyme.
**Lipoxygenase (LO) Assay:** An *in vitro* lipoxygenase (LO) inhibition assay. LOs are non-heme iron-containing dioxygenases that catalyze the addition of molecular oxygen to fatty acids. Linoleate and arachidonate are the main substrates for LOs in plants and animals. Arachadonic acid may then be converted to hydroxyeicosotrienenoic (HETE) acid derivatives, that are subsequently converted to leukotirenes, potent inflammatory mediators. This assay provides an accurate and convenient method for screening lipoxygenase inhibitors by measuring the hydroperoxides generated from the incubation of a lipoxygenase (5-, 12-, or 15-LO) with arachidonic acid. The Colorimetric LO Inhibitor screening kit (#760700, Cayman Chemical) can be used to determine the ability of each of the active ingredients, any one of the combination of ingredients, or compositions having said combinations disclosed in the specification to inhibit enzyme activity. Purified 15-lipoxygenase and test ingredients can be mixed in assay buffer and incubated with shaking for 10 min at room temperature. Following incubation, arachidonic acid can be added to initiate the reaction and mixtures incubated for an additional 10 min at room temperature. Colorimetric substrate can be added to terminate catalysis and color progression was evaluated by fluorescence plate reading at 490 nm. The percent inhibition of lipoxyganse activity can be calculated compared to non-treated controls to determine the ability of each of the active ingredients, any one of the combination of ingredients, or compositions having said combinations disclosed in the specification to inhibit the activity of purified enzyme.
**Elastase Assay:** EnzChek® Elastase Assay (Kit# E-12056) from Molecular Probes (Eugene, Oregon USA) can be used as an *in vitro* enzyme inhibition assay for measuring inhibition of elastase activity for each of the active ingredients, any one of the combination of ingredients, or compositions having said combinations disclosed in the specification. The EnzChek kit contains soluble bovine neck ligament elastin that can be labeled with dye such that the conjugate's fluorescence can be quenched. The non-fluorescent substrate can be digested by elastase or other proteases to yield highly fluorescent fragments. The resulting increase in fluorescence can be monitored with a fluorescence microplate reader. Digestion products from the elastin substrate have absorption maxima at ∼505 nm and fluorescence emission maxima at ∼515 nm. The peptide, chloromethyl ketone, can be used as a selective, collective inhibitor of elastase when utilizing the EnzChek Elastase Assay Kit for screening for elastase inhibitors.
**Oil Control Assay:** An assay to measure reduction of sebum secretion from sebaceous glands and/or reduction of sebum production from sebaceous glands can be assayed by using standard techniques known to those having ordinary skill in the art. In one instance, the forehead can be used. Each of the active ingredients, any one of the combination of ingredients, or compositions having said combinations disclosed in the specification can be applied to one portion of the forehead once or twice daily for a set period of days (*e.g.,* 1, 2, 3, 4, 5, 6, 7, 8, 9, 10, 11, 12, 13, 14, or more days), while another portion of the forehead is not treated with the composition. After the set period of days expires, then sebum secretion can be assayed by application of fine blotting paper to the treated and untreated forehead skin. This is done by first removing any sebum from the treated and untreated areas with moist and dry cloths. Blotting paper can then be applied to the treated and untreated areas of the forehead, and an elastic band can be placed around the forehead to gently press the blotting paper onto the skin. After 2 hours the blotting papers can be removed, allowed to dry and then transilluminated. Darker blotting paper correlates with more sebum secretion (or lighter blotting paper correlates with reduced sebum secretion.

### REFERENCES

The following references, to the extent that they provide exemplary procedural or other details supplementary to those set forth herein, are specifically incorporated herein by reference.
Cao *et al.* 1993.
International Cosmetic Ingredient Dictionary and Handbook, 12th Edition, 2008 ("CTFA"), Volume 2 page 2399
International Cosmetic Ingredient Dictionary and Handbook, 12th Edition, 2008 ("CTFA"), Volume 1 page 198, page 655
International Cosmetic Ingredient Dictionary and Handbook, 4th Edition, 1991 ("CTFA"), pp. 12 and 80

## Claims

1. A lipstick composition comprising:
color particles, wherein the color particles comprise a colorant, and wherein the colorant is selected from Blue 1 Lake, Red 6 Lake, Red 7 Lake, Yellow 5 Lake, iron oxides, titanium dioxide, tin oxide, and mica;
stearalkonium hectorite;
polyhydroxystearic acid;
polyglyceryl-10 pentaisostearate;
glyceryl behenate/eicosadioate;
isopropyl myristate;
tocopheryl acetate; and
a polar activator, wherein the polar activator is propylene carbonate;
wherein the composition further comprises microspheres that increase blendability of the composition, wherein the microspheres comprise HDI/Trimethylol hexyllactone crosspolymer and silica, wherein the composition comprises 0.5 to 15% by weight of HDI/Trimethylol hexyllactone crosspolymer and silica; wherein the color particles are uniformly dispersed throughout the composition.

2. The composition of claim 1, wherein the composition comprises:
0.01 to 0.1% by weight of stearalkonium hectorite;
0.001 to 0.1% by weight of polyhydroxystearic acid; and
0.001 to 0.1% by weight of a polar activator.

3. The composition of any one of claims 1 to 2, wherein the composition further comprises:
*Helianthus annus* (sunflower) seed oil;
pentaerythrityl tetraisostearate;
bis-diglycerylpolyacyladipate-2;
isononyl isononanoate;
polyethylene;
*Simmondsia chinensis* (jojoba) butter;
mica;
ozokerite;
sucrose polysoyate; and
microcrystalline wax/cire microcrystalline.

4. The composition of any one of claims 1 to 3 4 , wherein the composition further comprises triisostearyl citrate, in particular 7 to 13% by weight of triisostearyl citrate.

5. The composition of any one of claims 1 to 4 , wherein the composition comprises
2 to 10% by weight of polyglyceryl-10 pentaisostearate;
0.1 to 3% by weight of glyceryl behenate/eicosadioate;
0.1 to 3% by weight of isopropyl myristate; and
0.1 to 3% by weight of tocopheryl acetate.

6. The composition of any one of claims 1 to 5 , wherein the composition further comprises methylsilsesquioxane.

7. The composition of any one of claims 1 to , wherein the composition further comprises:
polyglyceryl-2 diisostearate/IPDI copolymer;
triisostearyl citrate;
DI-PPG-3 myristyl ether adipate; and
hydrogenated polyisobutene.

8. The composition of claim 7 , wherein the composition comprises
8 to 15% by weight of polyglyceryl-2 diisostearate/IPDI copolymer;
6 to 12% by weight of triisostearyl citrate;
2 to 10% by weight of DI-PPG-3 myristyl ether adipate; and
2 to 8% by weight of hydrogenated polyisobutene.

9. The composition of any one of claims 1 to 8 , wherein the composition comprises 0.5 to 3%, 1 to 3%, 2 to 6%, or 9 to 13% by weight of HDI/Trimethylol hexyllactone crosspolymer.

10. The composition of any one of claims 1 to 9 , wherein the composition is anhydrous.

11. The composition of any one of claims 1 to 10 wherein the composition further comprises one or more additional ingredients selected from one or more colorants, bulking agents, thickening agents, emulsifiers, fragrances, moisturizing agents, conditioning agents, and antioxidants.

12. The composition of any one of claims 1 to 11 , wherein the composition is semi-solid.

13. A method for coloring or moisturizing lips comprising applying the composition of any one of claims 1 to 12 to the lips.

## Patentansprüche

1. Lippenstift-Zusammensetzung, umfassend:
Farbteilchen, wobei die Farbteilchen ein Farbmittel umfassen, und wobei das Farbmittel ausgewählt ist aus Blau 1 See, Rot 6 See, Rot 7 See, Gelb 5 See, Eisenoxiden, Titandioxid, Zinnoxid und Glimmer,
Stearalkoniumhektorit,
Polyhydroxystearinsäure,
Polyglyceryl-10-Pentaisostearat,
Glycerylbehenat/eicosadioat,
Isopropylmyristat,
Tocopherylacetat, und
einen polaren Aktivator, wobei der polare Aktivator Propylencarbonat ist,
wobei die Zusammensetzung weiterhin Mikrokugeln umfasst, die die Mischbarkeit der Zusammensetzung erhöhen, wobei die Mikrokugeln HDI/Trimethylolhexyllacton-Crosspolymer und Siliciumdioxid umfassen, wobei die Zusammensetzung 0,5 bis 15 Gew.-% HDI/Trimethylolhexyllacton-Crosspolymer und Siliciumdioxid umfasst, wobei die Farbteilchen gleichmäßig in der Zusammensetzung dispergiert sind.

2. Die Zusammensetzung nach Anspruch 1, wobei die Zusammensetzung umfasst:
0,01 bis 0,1 Gew.-% Stearalkoniumhektorit,
0,001 bis 0,1 Gew.-% Polyhydroxystearinsäure, und
0,001 bis 0,1 Gew.-% polaren Aktivator.

3. Die Zusammensetzung nach einem der Ansprüche 1 bis 2, wobei die Zusammensetzung weiterhin umfasst:
*Helianthus annus* (Sonnenblumen)-Samenöl,
Pentaerythrittetraisostearat,
Bis-Diglycerylpolyacyladipat-2,
Isononylisononanoat,
Polyethylen,
*Simmondsia chinensis* (Jojoba)-Butter,
Glimmer,
Ozokerit,
Saccharosepolysoyat, und
mikrokristallines Wachs.

4. Zusammensetzung nach einem der Ansprüche 1 bis 3, wobei die Zusammensetzung ferner Triisostearylcitrat, insbesondere 7 bis 13 Gew.-% Triisostearylcitrat, umfasst.

5. Die Zusammensetzung nach einem der Ansprüche 1 bis 4, wobei die Zusammensetzung umfasst
2 bis 10 Gew.-% Polyglyceryl-10-pentaisostearat,
0,1 bis 3 Gew.-% Glycerylbehenat/Eicosadioat,
0,1 bis 3 Gew.-% Isopropylmyristat, und
0,1 bis 3 Gew.-% Tocopherylacetat.

6. Die Zusammensetzung nach einem der Ansprüche 1 bis 5, wobei die Zusammensetzung weiterhin Methylsilsesquioxan umfasst.

7. Die Zusammensetzung nach einem der Ansprüche 1 bis 6, wobei die Zusammensetzung weiterhin umfasst:
Polyglyceryl-2-diisostearat/IPDI-Copolymer,
Triisostearylcitrat,
DI-PPG-3-Myristylätheradipat, und
hydriertes Polyisobuten.

8. Die Zusammensetzung nach Anspruch 7, wobei die Zusammensetzung umfasst
8 bis 15 Gew.-% Polyglyceryl-2-diisostearat/IPDI-Copolymer,
6 bis 12 Gew.-% Triisostearylcitrat,
2 bis 10 Gew.-% DI-PPG-3-Myristyletheradipat, und
2 bis 8 Gew.-% hydriertes Polyisobuten.

9. Zusammensetzung nach einem der Ansprüche 1 bis 8, wobei die Zusammensetzung 0,5 bis 3 Gew.-%, 1 bis 3 Gew.-%, 2 bis 6 Gew.-% oder 9 bis 13 Gew.-% HDI/Trimethylolhexyllacton-Crosspolymer umfasst.

10. Die Zusammensetzung nach einem der Ansprüche 1 bis 9, wobei die Zusammensetzung wasserfrei ist.

11. Zusammensetzung nach einem der Ansprüche 1 bis 10, wobei die Zusammensetzung weiterhin einen oder mehrere zusätzliche Inhaltsstoffe, ausgewählt aus einem oder mehreren Farbstoffen, Füllstoffen, Verdickungsmitteln, Emulgatoren, Duftstoffen, Feuchthaltemitteln, Konditionierungsmitteln und Antioxidantien, umfasst.

12. Die Zusammensetzung nach einem der Ansprüche 1 bis 13, wobei die Zusammensetzung halbfest ist.

13. Verfahren zum Färben oder Befeuchten der Lippen, bei dem die Zusammensetzung nach einem der Ansprüche 1 bis 12 auf die Lippen aufgetragen wird.

## Revendications

1. Composition de rouge à lèvres comprenant :
des particules de couleur, où les particules de couleur comprennent un colorant, et où le colorant est choisi parmi les Blue 1 Lake, Red 6 Lake, Red 7 Lake, Yellow 5 Lake, les oxydes de fer, le dioxyde de titane, l'oxyde d'étain, et le mica ;
de l'hectorite de stéaralkonium ;
du poly(acide hydroxystéarique) ;
du pentaisostéarate de polyglycéryle-10 ;
du béhénate/éicosadioate de glycéryle ;
du myristate d'isopropyle ;
de l'acétate de tocophéryle ; et
un activateur polaire, où l'activateur polaire est le carbonate de propylène ;
où la composition comprend en outre des microsphères qui augmentent l'aptitude à la combinaison de la composition, où les microsphères comprennent un polymère réticulé de HDI/triméthylol-hexyl-lactone et de la silice, où la composition comprend 0,5 à 15 % en poids de polymère réticulé de HDI/triméthylol-hexyl-lactone et de silice ; où les particules de couleur sont dispersées uniformément dans toute la composition.

2. Composition selon la revendication 1, où la composition comprend :
0,01 à 0,1 % en poids d'hectorite de stéaralkonium ;
0,001 à 0,1 % en poids de poly(acide hydroxystéarique) ; et
0,001 à 0,1 % en poids d'un activateur polaire.

3. Composition selon l'une quelconque des revendications 1 et 2, où la composition comprend en outre :
de l'huile de graines de *Helianthus annus* (tournesol) ;
du tétraisostéarate de pentaérythrityle ;
du polyacyladipate-2 de bis-diglycéryle ;
de l'isononanoate d'isononyle ;
du polyéthylène ;
du beurre de *Simmondsia chinensis* (jojoba) ;
du mica ;
de l'ozokérite ;
du poly(soja-yl)ate de saccharose ; et
de la cire microcristalline.

4. Composition selon l'une quelconque des revendications 1 à 3, où la composition comprend en outre du citrate de triisostéaryle, en particulier 7 à 13 % en poids de citrate de triisostéaryle.

5. Composition selon l'une quelconque des revendications 1 à 4, où la composition comprend
2 à 10 % en poids de pentaisostéarate de polyglycéryle-10 ;
0,1 à 3 % en poids de béhénate/éicosadioate de glycéryle ;
0,1 à 3 % en poids de myristate d'isopropyle ; et
0,1 à 3 % en poids d'acétate de tocophéryle.

6. Composition selon l'une quelconque des revendications 1 à 5, où la composition comprend en outre du méthylsilsesquioxane.

7. Composition selon l'une quelconque des revendications 1 à 6, où la composition comprend en outre :
un copolymère de diisostéarate de polyglycéryle-2/IPDI ;
du citrate de triisostéaryle ;
de l'adipate d'éther myristylique de DI-PPG-3 ; et
du polyisobutène hydrogéné.

8. Composition selon la revendication 7, où la composition comprend
8 à 15 % en poids de copolymère de diisostéarate de polyglycéryle-2/IPDI ;
6 à 12 % en poids de citrate de triisostéaryle ;
2 à 10 % en poids d'adipate d'éther myristylique de DI-PPG-3 ; et
2 à 8 % en poids de polyisobutène hydrogéné.

9. Composition selon l'une quelconque des revendications 1 à 8, où la composition comprend 0,5 à 3 %, 1 à 3 %, 2 à 6 %, ou 9 à 13 % en poids de polymère réticulé de HDI/triméthylol-hexyl-lactone.

10. Composition selon l'une quelconque des revendications 1 à 9, où la composition est anhydre.

11. Composition selon l'une quelconque des revendications 1 à 10, où la composition comprend en outre un ou plusieurs ingrédients additionnels choisis parmi un ou plusieurs colorants, agents de charge, agents épaississants, émulsionnants, parfums, agents hydratants, agents conditionneurs, et antioxydants.

12. Composition selon l'une quelconque des revendications 1 à 11, où la composition est semi-solide.

13. Procédé pour colorer ou hydrater les lèvres, comprenant l'application sur les lèvres de la composition de l'une quelconque des revendications 1 à 12.
